# EUROPÄISCHE PATENTSCHRIFT

(11) **EP 1 943 987 B1**
(45) Veröffentlichungstag und Bekanntmachung des Hinweises auf die Patenterteilung: **18.04.2012**
(21) Anmeldenummer: 08101635.4
(22) Anmeldetag: 26.10.2005
(51) Int. Cl.: A61F 2/44, A61B 17/70

(54) **Implantat mit einstückigem Drehgelenk**
Implant with one-piece swivel joint
Implant doté d'une articulation tournante en une pièce

(43) Veröffentlichungstag der Anmeldung: 16.07.2008
(62) Teilanmeldung aus: 05110043.6
(73) Patentinhaber: BIEDERMANN MOTECH GmbH, 78054 VS-Schwenningen (DE)
(72) Erfinder: Biedermann, Lutz, 78048, VS-Villingen (DE); Matthis, Wilfried, 79367, Weisweil (DE); Harms, Jürgen, 76227, Karlsruhe (DE)
(74) Vertreter: Lang, Christian

(56) Entgegenhaltungen:
- EP-A- 0 538 183
- EP-A- 0 950 389
- EP-A2- 0 677 277
- WO-A2-2004/105577
- FR-A1- 2 775 587
- US-A- 6 136 031
- US-A1- 2001 016 774
- US-A1- 2005 131 405
- US-B1- 6 579 321

## Beschreibung

### Technisches Gebiet

Die vorliegende Erfindung betrifft ein Implantat zur dauerhaften oder zeitweisen Verbringung in den menschlichen oder tierischen Körper gemäß dem Oberbegriff des Anspruchs 1 bzw. einen Platzhalter für Wirbelkörper, einen Bandscheibenersatz oder einen Verbindungsstab für Pedikelschraubenanordnungen sowie ein Stabilisierungssystem aus diesen Komponenten.

### Stand der Technik

In der modernen Medizin werden seit geraumer Zeit vielfältig implantate verwendet, um nach Krankheiten, Verletzungen oder altersbedingten Abnutzungserscheinungen Teile des menschlichen Skeletts zu ersetzen. So ist es beispielsweise bekannt, bei Wirbelsäulenverletzungen, wie Brüchen oder bei einem Angriff von Teilen der Wirbelsäule durch einen Tumor, entsprechende Wirbelkörper durch Platzhalter zu ersetzen. Derartige Platzhalter sind beispielsweise in der europäischen Patentschrift EP 0 268 115 B1 beschrieben.

In ähnlicher Weise können auch Bandscheiben durch entsprechende Platzhalter ersetzt werden, wie beispielsweise in der DE 43 23 034 C1 beschrieben. Diese Platzhalter, die im Wesentlichen eine zylinderrohrförmige Grundstruktur aufweisen, müssen im Wesentlichen axiale Druckkräfte aufnehmen, um die Last, die von der Wirbelsäule getragen wird, abzuleiten. Hierzu ist eine ausreichende Festigkeit der Platzhalter erforderlich.

Darüber hinaus ist es jedoch wünschenswert, dass die Platzhalter eine gewisse Flexibilität aufweisen, um Bewegungen der Wirbelsäule, insbesondere Verbiegungen oder Verdrehungen mitzumachen. Zu diesem Zweck sind nach dem Stand der Technik Platzhalter für Wirbelkörper oder Bandscheiben vorgeschlagen worden, die eine Kombination der beiden Funktionalitäten, nämlich einerseits Festigkeit, insbesondere in axialer Druckrichtung, und andererseits Beweglichkeit, insbesondere Biegbarkeit um eine Drehachse senkrecht zur Achslastrichtung, realisieren. Beispiele hierfür sind die DE 103 37 088 A1 sowie die WO 2005/039454 A2. Bei diesen Platzhaltern wird im Mittelbereich zwischen den Enden, die zur Anordnung oder Befestigung im benachbarte Gewebe oder benachbarten Wirbeln dienen, ein flexibler Bereich vorgesehen, der entweder durch entsprechende elastische Werkstoffe oder durch eine entsprechende Struktur des Platzhalters erreicht wird. Die WO 2005/039454 schlägt hierzu vor, einen spiralförmig umlaufenden Schlitz vorzusehen, der dem Grundkörper im elastischen Mittelbereich eine federartige Wirkung verleiht.

Die WO 2005/039454 A2 offenbart weiterhin, dass auch andere Implantate, wie z.B. Verbindungsstäbe für Pedikelschraubenanordnungen oder ähnliche Stabilisierungssysteme, durch entsprechende Helix-Formen neben der Fähigkeit Kräfte zu übertragen auch eine gewisse Flexibilität aufweisen können. Ein Implantat gemäß dem Oberbegriff des Anspruchs 1 wird in US 2001/0016774 offenbart.

Obwohl mit diesen Lösungen sehr gute Ergebnisse hinsichtlich der erzielbaren Flexibilität erreicht werden, sind durch die Unbestimmtheit der Dreh- oder Biegeachsen derartiger Strukturen Defizite bezüglich der Festigkeit und der aufnehmbaren axialen Drucklast in Kauf zu nehmen.

### Darstellung der Erfindung

### Technische Aufgabe

Es ist deshalb Aufgabe der vorliegenden Erfindung eine weitere Verbesserung des Eigenschaftsprofils hinsichtlich der an sich gegenläufigen Funktionalitäten, nämlich Flexibilität bzw. Beweglichkeit einerseits und Festigkeit bzw. Lastaufnahmefähigkeit andererseits, zu erzielen, wobei insgesamt das Implantat bzw. insbesondere der Platzhalter bei der Operation einfach einsetzbar und im übrigen einfach herstellbar sein soll.

### Technische Lösung

Diese Aufgabe wird gelöst mit einem Implantat mit den Merkmalen des Anspruchs 1. Vorteilhafte Ausgestaltungen sind Gegenstand der abhängigen Ansprüchen.

Die vorliegende Erfindung geht aus von der Erkenntnis, dass zur Verbesserung eines ausgewogenen Verhältnisses von Flexibilität bzw. Beweglichkeit einerseits und Steifigkeit bzw. Festigkeit und Lastaufnahmefähigkeit andererseits definierte Drehgelenke vorgesehen werden sollten, die einerseits eine Fähigkeit zur Lastübertragung, beispielsweise in axialer Lastrichtung, aufweisen, aber andererseits eine Drehung um die Drehachse des definierten Drehgelenks und somit Biegung des Implantats ermöglichen.

Dies wird insbesondere dann möglich, wenn das Drehgelenk einstückig in dem Implantat ausgebildet wird, was hier durch ein so genanntes Filmscharnier verwirklicht wird. Filmscharnier bedeutet hier, dass ein dünner Film- bzw. Stegbereich oder allgemein ein Wandbereich vorgesehen ist, der durch die dimensionale Auslegung eine entsprechende Elastizität und damit Drehbewegung bzw. Schwenkbewegung ermöglicht, obwohl der entsprechende Wandbereich oder die entsprechenden Stege aus dem gleichen, insbesondere an sich steifen Material gefertigt sind, wie die übrigen Bereiche.

Erfindungsgemäß sind derartige Drehgelenke vorgesehen, um ein Verkippen der Enden des Implantats bzw. eine Verbiegung zu ermöglichen. Diese Drehgelenke sind in verschiedenen Ebenen, insbesondere entlang der Hauptlastachse sowie verdreht um die Hauptlastachse angeordnet. Dabei sind verschiedene Drehachsen in verschiedenen Ebenen alternatierend um 90° zueinander versetzt, so dass eine Verbiegung des Implantats in jede Richtung gewährleistet wird.

Das Implantat ist so gestaltet, dass der rohrförmige, insbesondere zylinderrohrförmige Grundkörper aus mehreren übereinander angeordneten Scheiben- bzw. Ringelementen aufgebaut ist, die jeweils durch entsprechende Drehgelenk bzw. Filmscharniere miteinander verbunden sind, im übrigen aber beabstandet zueinander angeordnet sind, so dass ein freier Bewegungsweg bei der Drehung um das entsprechende Drehgelenk vorhanden ist.

Die Drehgelenke sind jeweils entlang der Halbierenden der einzelnen Ring- bzw. Scheibenelemente angeordnet, während sich beidseits des Filmscharniers eine Aussparung anschließt, die den notwendigen Bewegungsraum zur Verfügung stellt.

Hier ist es vorteilhaft, wenn die Aussparung bzw. der Freiraum zwischen benachbarten Ring- bzw. Scheibenelementen, d.h. der Abstand zwischen diesen, ausgehend von dem Drehgelenk bzw. Filmscharnier abnimmt, so dass am Rand lediglich ein schlitzförmiger Abstand gegeben ist. Dieser schlitzförmige Abstand definiert einerseits die mögliche Verkippung benachbarter Scheiben- bzw. Ringelemente zueinander und definiert gleichzeitig bei einer axialen Stauchung entlang der Hauptlastachse des Implantats die potentiellen gegenseitigen Auflageflächen, die zu einer Lastabtragung dienen können. Dies führt dazu, dass bei einer axialen Druckbelastung entlang der Hauptlastachse zunächst das Drehgelenk bzw. Filmscharnier die Last aufnimmt. Durch die Ausgestaltung der über die Filmscharniere untereinander verbundenen Scheiben oder Ringe mit einer auf Grund der größeren Ausnehmungen geringeren Dicke der Scheiben oder Ringe in der Nähe der Filmscharniere, kommt es bei weiter zunehmender axialer Belastung zu einer elastischen Verformung bzw. insbesondere Verbiegung der Scheiben bzw. Ringe bis der schlitzförmige Abstand zwischen den benachbarten Scheiben- bzw. Ringelementen. aufgebraucht ist. Sobald die benachbarten Scheiben- bzw. Ringelemente im Bereich der schlitzförmigen Ausnehmung aufeinander liegen, übernehmen diese Randbereiche der rohrförmigen Grundstruktur ebenfalls die Funktion der Lastabtragung, so dass auch bei sehr hohen axialen Drucklasten eine entsprechende Festigkeit vorhanden ist. Gleichzeitig wird aber durch die Mikrobewegung, die durch die schlitzförmigen Ausnehmungen sowohl in axialer Richtung z. B. durch Stauchung oder durch Drehung oder Biegung quer zur Hauptlastachse gewährleistet wird, eine Überlastung der Nachbarsegmente verhindert und ein schnelles Anwachsen der Endplatten eines entsprechenden Platzhalters erleichtert, da dieser kleine Bewegungen mitmacht, ohne dass es zu einem Abreißen an den Endplatten kommen würde.

Entsprechend sind vorzugsweise an den Endplatten bzw. Endscheiben bzw. -ringen Verbindungsmittel vorgesehen, die ein Eingreifen und Einwachsen in benachbarte Körperteile bzw. eine Anbringung des Implantats an andere Implantatkomponenten ermöglicht. Hierzu sind Zacken, abgestumpfte Zacken, Vertiefungen, Ausnehmungen und dergleichen vorgesehen, die sich insbesondere auch dadurch auszeichnen, dass sie durch ihre Gestaltung leicht auf eine gewünschte Länge angepasst werden können.

Die Filmscharniere werden so gebildet, dass ausgehend von der äußeren Umfangswand des rohrförmigen Grundkörpers zwischen den benachbarten Scheiben- bzw. Ringelementen radial nach innen verlaufende Stege vorgesehen sind, die zusätzlich durch seitliche Plattenelemente mit den entsprechenden Scheiben- bzw. Ringelementen verbunden werden können. Vorzugsweise werden die Stege bzw. Filmscharniere nicht durchgehend ausgeführt, sondern es werden zwei gegenüberliegende Stege vorgesehen, die im zentralen Bereich beabstandet zueinander angeordnet sind, so dass sich entlang der Mittelachse eine durchgehende Öffnung ergibt, die unterschiedlich in ihrer Form und Struktur gestaltet sein kann, beispielsweise kleeblattförmige, kreuzförmig oder dergleichen.

Das Material für den vorzugsweise einstückig gefertigten Grundkörper also insbesondere die Scheiben und Ringe bzw. Stege der Filmscharniere kann aus unterschiedlichen Materialen gewählt werden. Insbesondere sind hier alle biokompatiblen Metalle, Metalllegierungen oder Kunststoffe geeignet. Durch die erfindungsgemäße Struktur des Grundkörpers können auch an sich sehr steife Materialien eingesetzt werden, da die Flexibilität und Beweglichkeit des Grundkörpers durch die konstruktive Gestaltung gewährleistet ist. Selbstverständlich können jedoch auch Werkstoffe eingesetzt werden, die von Haus aus eine gewisse Elastizität aufweisen und damit die Beweglichkeit und Flexibilität gewährleisten.

### Kurze Beschreibung der Zeichnungen

Weiter Vorteile, Kennzeichen und Merkmale der vorliegenden Erfindung werden bei der nachfolgenden detaillierten Beschreibung von Ausführungsbeispielen anhand der beigefügten Zeichnungen deutlich. Diese zeigen in rein schematische Weise in

Fig. 1 eine perspektivische Darstellung eines erfindungsgemäßen Platzhalters;

Fig. 2 eine perspektivische Darstellung eines zweiten erfindungsgemäßen Platzhalters;

Fig. 3 eine perspektivische Darstellung eines dritten erfindungsgemäßen Platzhalters;

Fig. 4 eine Seitenansicht des Platzhalters aus Fig. 4;

Fig. 5 eine Seitenansicht des Platzhalters aus den Fig. 1 und 4, wobei die Seitenansicht der Fig. 5 um 90° gegenüber derjenigen der Fig. 4 gedreht ist;

Fig. 6 eine Seitenansicht des Platzhalters aus Fig. 3;

Fig. 7 eine Seitenansicht des Platzhalters aus den Fig. 3 und 6, wobei die Seitenansicht des Platzhalters in Fig. 7 um 90° gegenüber derjenigen aus Fig. 6 verdreht ist;

Fig. 8 eine Draufsicht auf den Platzhalter aus Fig. 3;

Fig. 9 eine Draufsicht auf den Platzhalter aus Fig. 1;

Fig. 10 eine Seitenansicht eines Teils der vorher beschriebenen Platzhalter in unterschiedlichen Belastungszuständen in den Teilbildern a) bis c);

Fig. 11 eine Seitenansicht eines Teils des Platzhalters der vorangegangenen Beispiele in Seitenansicht mit einer in den Teilbildern a) bis c) unterschiedlichen Belastung;

Fig. 12 eine Detailansicht des Platzhalters aus Fig. 1;

Fig. 13 eine seitliche Schemadarstellung des Platzhalters aus Fig. 1 im Zusammenspiel mit einer Pedikelschraubenanordnung;

Fig. 14 eine seitliche Schemadarstellung des Zusammenwirkens des Platzhalters aus Fig. 1 mit einer weiteren Form einer Pedikelschraubenanordnung;

Fig. 15 eine schematische Seitenansicht des Einsatzes des Platzhalters aus Fig. 3;

Fig. 16 eine seitliche Schemadarstellung eines weiteren Einsatzes des Platzhalters aus Fig. 3 im Zusammenwirken mit einer Pedikelschraubenanordnung;

Fig. 17 eine seitliche Schemadarstellung eines erfindungsgemäßen Verbindungsstabs;

Fig. 18 eine Detailansicht des erfindungsgemäßen Verbindungsstabs aus Fig. 17 in einer teilweisen Schnittansicht;

Fig. 19 eine Schnittansicht des Verbindungsstabes aus Fig. 17 und 18;

Fig. 20 eine perspektivische Darstellung des erfindungsgemäßen Verbindungsstabs aus den Fig. 17 - 19;

Fig. 21 eine seitliche Schemadarstellung der Verwendung des erfindungsgemäßen Platzhalters aus Fig. 1 und des erfindungsgemäßen Verbindungsstabes aus Fig. 20.

### Weg(e) zur Ausführung der Erfindung

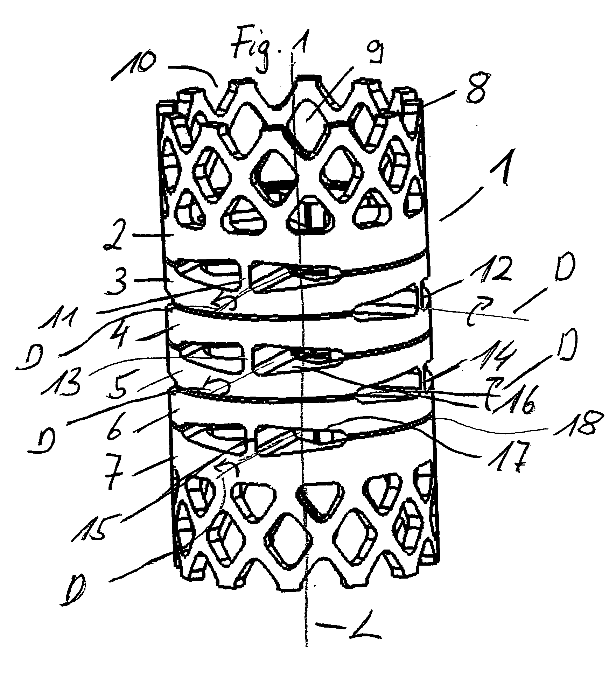

Die Fig. 1 zeigt in einer perspektivischen Darstellung eine erste Ausführungsform eines erfindungsgemäßen Implantats in Form eines Platzhalters für die Wirbelsäule.

Der Platzhalter 1 weist einen im Wesentlichen zylinderrohrförmigen Grundkörper auf, der in sechs Scheiben bzw. Ringe 2 bis 7 unterteilt ist, die entlang der Längsachse L, die zugleich die primäre Lastachse L ist, übereinander angeordnet sind. Die beiden Endelemente 2 und 7 weisen an den nach außen weisenden Enden Verbindungselemente 8 bis 10 auf, die aus stumpfen Zacken, rautenförmigen bzw. dreiecksförmigen Ausnehmungen 9 und dreiecksförmigen Vertiefungen 10 zwischen den stumpfen Zacken gebildet sind. Diese Verbindungselemente 8 bis 10 dienen zum Eingreifen und Verwachsen mit benachbartem Gewebe, Knorpeln oder Wirbelkörpern. Mit den Verbindungselementen 8 bis 10 wird eine sichere Anordnung des Platzhalters 1 in der Wirbelsäule gewährleistet.

Zwischen den Scheiben- bzw. Ringelementen 2 bis 7 sind jeweils zwei von außen radial nach innen sich erstreckende Stege vorgesehen, die die Filmscharniere 11 bis 15 bilden. Die die Filmscharniere 11 bis 15 bildenden Stege verlaufen nicht komplett bis zur Mittelachse, welche parallel bzw. identisch zur Lastachse L ist, sondern sind im Innenbereich beabstandet zueinander, so dass sich eine durchgehende Öffnung bildet, die später noch erläutert wird. Alternativ können die Stege auch auf eine Breite entsprechend der Wanddicke des Grundkörpers bzw. der Ringe 2 bis 7 beschränkt sein.

Darüber hinaus sind die Filmscharniere abwechselnd um 90° verdreht, so dass das Filmscharnier 11 senkrecht zum Filmscharnier 12 angeordnet ist, während dieses wiederum senkrecht zum Filmscharnier 13 angeordnet ist usw..

Die Scheiben bzw. Ringelemente 2 bis 7 sind jeweils nur durch die entsprechenden Filmscharniere 11 bis 15 miteinander verbunden, die zwei halbkreisförmige Ausnehmungen zwischen den entsprechenden Scheiben bzw. Ringelementen 2 bis 7 voneinander trennen. Die entsprechenden Ausnehmungen weisen im Bereich der Filmscharniere 11 bis 15 eine größere Dicke in Richtung der Lastachse L auf als in weiter von den Filmscharnieren 11 bis 15 entfernten Bereichen. Insbesondere sind die Ausnehmungen zwischen den Scheiben- bzw. Ringelementen 2 bis 7 zweigeteilt, wobei der erste Teil eine im Querschnitt dreiecksförmige Ausnehmung bildet, die ausgehend von den Stegen der Filmscharniere 11 bis 15 mit einer großen Dicke in Richtung der Lastachse mit zunehmendem Abstand von den Filmscharnieren 11 bis 15 in der Dicke abnimmt und schließlich in einem zweiten Teil in einen dünnen Schlitz 18 zwischen den Scheiben- bzw. Ringelementen 2 bis 7 ausläuft. Die im Wesentlichen im Querschnitt dreiecksförmige Gestalt der Ausnehmung 17 ist durch abgerundete Kanten dahingehend optimiert, dass keine Spannungsspitzen auftreten.

Durch die beidseitig der Filmscharniere vorgesehenen Ausnehmungen 17, 18 wird eine Verkippung des Platzhalters 1 um eine Drehachse parallel zu den Stegen der Filmscharniere 11 bis 15 ermöglicht, wie dies durch die Drehachsen D und die entsprechenden Drehpfeile angedeutet ist.

Durch die um 90° versetzte Anordnung der Filmscharniere 11, 13 und 15 zu den Filmscharnieren 12 und 14 ist eine Verkippung bzw. Verbiegung des Platzhalters 1 in jede Richtung um die Lastachse L möglich, wobei saggitale Verkippungswinkel im Bereich von bis zu 5° erreicht werden.

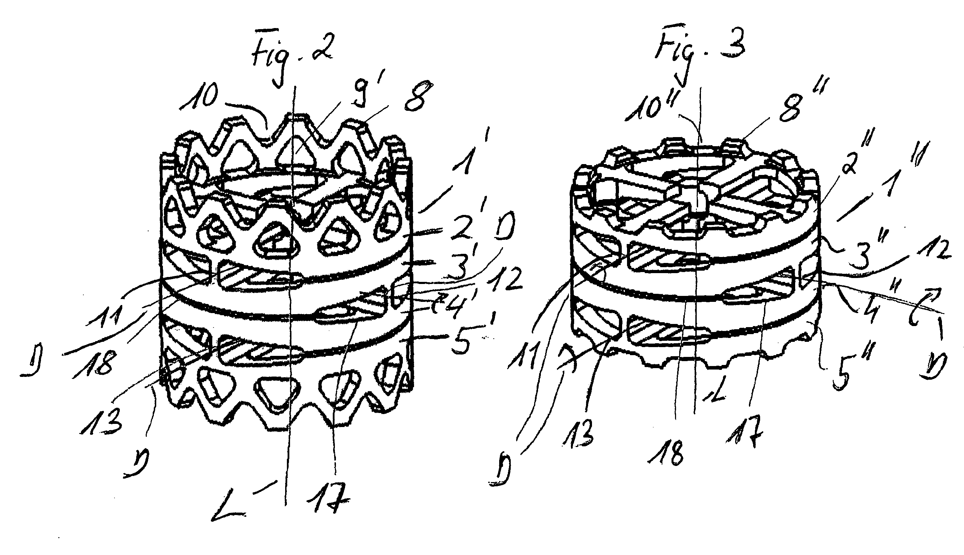

Zwei weitere Ausführungsformen eines Platzhalters 1' und 1" sind in perspektivischen Darstellungen der Fig. 2 und 3 gezeigt, wobei identische Komponenten mit identischen Bezugszeichen bezeichnet sind.

Der Platzhalter 1' weist entlang der Lastachse L, zeitlang der primär Zug-und Druckkräfte von dem Platzhalter l'aufgenommen werden, vier Scheiben- bzw. Ringelemente 2', 3', 4' und 5' auf. Diese sind miteinander über Drehgelenke bzw. Filmscharniere 11, 12 und 13 verbunden, wobei diese jeweils Drehachsen D senkrecht zur Lastachse L bilden.

Benachbart zu den Filmscharnieren 11, 12 und 13 sind jeweils beidseitig von den Stegen der Filmscharniere 11 bis 13 im Querschnitt im wesentlichen dreiecksförmige Ausnehmungen 17 vorgesehen, die sich mit zunehmendem Abstand von den Filmscharnieren 11 bis 13 verjüngen und in dünne Schlitze 18 auslaufen.

An den in Richtung der Lastachse L angeordneten Enden, nämlich den Ringelementen 2' und 5' sind Verbindungselemente zur Verbindung mit benachbarten Wirbelkörpern, Knorpeln oder Gewebe vorgesehen, wobei identisch zur Ausführungsform der Fig. 1 abgestumpfte Zacken 8 und dreiecksförmige Ausnehmungen 10 vorgesehen sind. Darüber hinaus sind lediglich dreiecksförmige Ausnehmungen 9' vorhanden, da der Kranz an Verbindungselementen des Platzhalters 1' deutlich niedriger ausgebildet ist als derjenige des Platzhalters 1.

Eine weitere Verringerung des Kranzes an Verbindungselementen ist bei dem Platzhalter 1 ", der in Fig. 3 dargestellt ist, festzustellen. Hier sind keinerlei Ausnehmungen mehr vorgesehen, sondern lediglich abgestumpfte Spitzen 8" und trapezförmige Vertiefungen 10".

Im übrigen sind auch hier vier Scheibenelemente 2", 3", 4" und 5" über alternierend, senkrecht zueinander angeordnete Filmscharniere 11, 12 und 13 miteinander verbunden, während im übrigen zwischen den Scheibenelementen 2", 3", 4" und 5" entsprechende Ausnehmungen vorhanden sind, die eine beabstandete Anordnung der Scheibenelemente 2", 3", 4" und 5" in den Bereichen außerhalb der Filmscharnier bewirken. Der Abstand zwischen den Schreibenelementen 2", 3", 4" und 5" ist im Bereich der im Querschnitt dreiecksförmigen Ausnehmung 17 deutlich größer als im Bereich der Schlitzausbildung 18, wobei hier ebenso wie bei den anderen Ausführungsbeispielen ungefähr die Hälfte des Umfangs im Bereich des Schlitzabstandes vorgesehen ist, während die andere Hälfte des Umfangs einen größeren Abstand zwischen den Scheibenelementen 2" bis 5" aufweist.

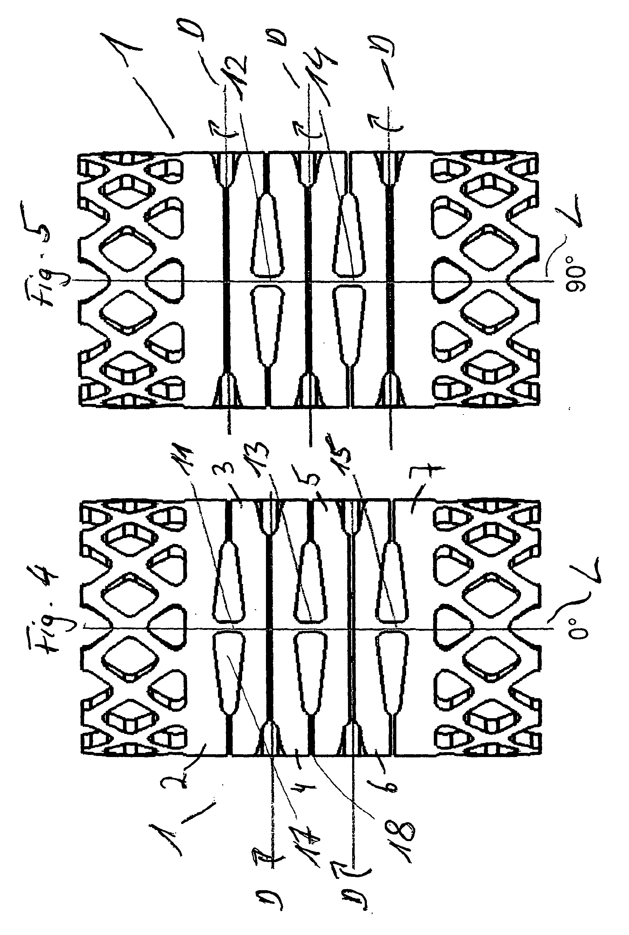

Die Figuren 4 bis 7 verdeutlichen noch einmal das konstruktive Prinzip der erfindungsgemäßen Implantats anhand der Platzhalter 1 und 1 ". In der Figuren 4 und 5 sind zwei Seitenansichten des Platzhalters 1 dargestellt, die um 90° um die Achse L zueinander verdreht sind. Deutlich ist im Vergleich der Figuren 4 und 5 zu erkennen, dass die Filmscharniere 11, 13 und 15 einerseits sowie die Filmscharniere 12 und 14 andererseits sowie die entsprechenden zugehörigen Drehachsen D um 90° verdreht zueinander angeordnet sind, wobei durch die Schlitze 18 zwischen den entsprechenden Ring- bzw. Scheibenelementen 2 bis 7 eine Verkippung um die jeweiligen Drehachsen D möglich ist.

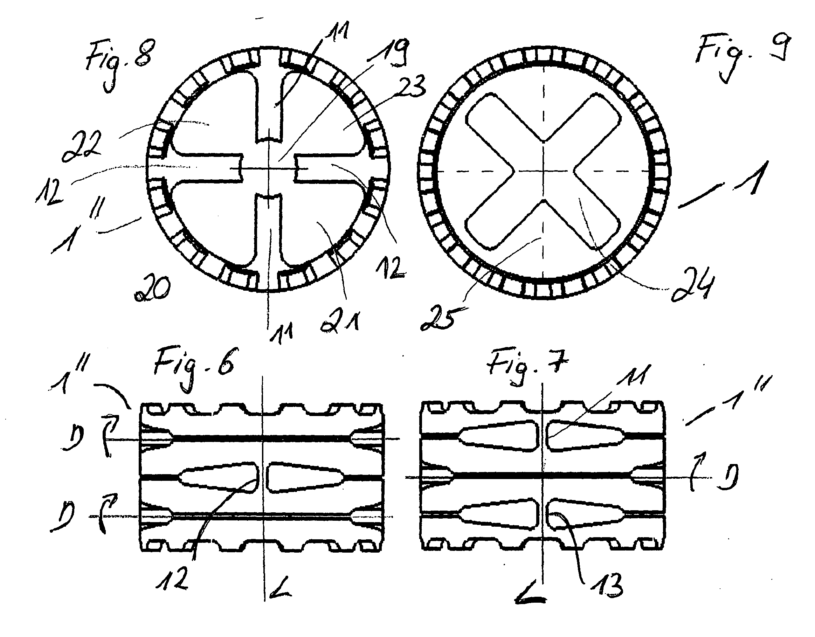

Das gleiche ist in den Figuren 6 und 7 für den Platzhalter 1" mit einer verringerten Anzahl von Scheiben- bzw. Ringelementen sowie Filmscharnieren 11 bis 13 und Drehachsen D gezeigt.

Die Figuren 8 und 9 zeigen Draufsichten auf die Platzhalter 1" (Figur 8) bzw. 1 (Fig. 9). Aus diesen Draufsichten wird deutlich, dass sowohl der Platzhalter 1" als auch der Platzhalter 1 einen im wesentlichen zylinderrohrförmigen Körper aufweisen, der durch die entsprechenden Ring- bzw. Scheibenelemente aufgebaut ist. Durch die Draufsichten wird ebenfalls deutlich, dass die Filmscharniere, beispielsweise Filmscharniere 11 und 12 des Platzhalters 1" in der Fig. 8, durch radial nach innen verlaufende Stege gebildet sind, die in der Mitte jedoch beabstandet zueinander ausgeführt sind, so dass dort eine zentrale Öffnung 19 durch den gesamten Platzhalter 1" entlang der Lastachse L gebildet ist.

Sofern die Stege der Filmscharniere 11 bis 15 lediglich über die Außenwand der verbundenen Ring- bzw. Scheibenelemente befestigt sind, ergeben sich zwischen den entsprechenden Stegen der Filmscharniere 11 bis 15 weitere Hohlräume 20 bis 23, die ebenfalls parallel zur Lastachse durchgehend sind, so dass sich insgesamt von oben bzw. unten ein kleeblattförmiger Freiraum 19 bis 23 beim Platzhalter 1 " ergibt.

Alternativ können die die Filmscharniere bildenden Stege jedoch auch seitlich durch entsprechende Platten 16 (siehe Fig. 1) stabilisiert bzw. an den entsprechenden Ring- bzw. Scheibenelementen 2 bis 7 angebunden sein. Sind derartige Platten 16, wie bei der Ausführungsform der Fig. 1, dreiecksförmig ausgebildet, so ergibt sich insgesamt in der Draufsicht ein kreuzförmiger durchgehender Hohlraum 24, wobei die die Filmscharniere 11 bis 15 bildenden Stege im Bereich der Strichlinien 25 der Fig. 9 angeordnet sind.

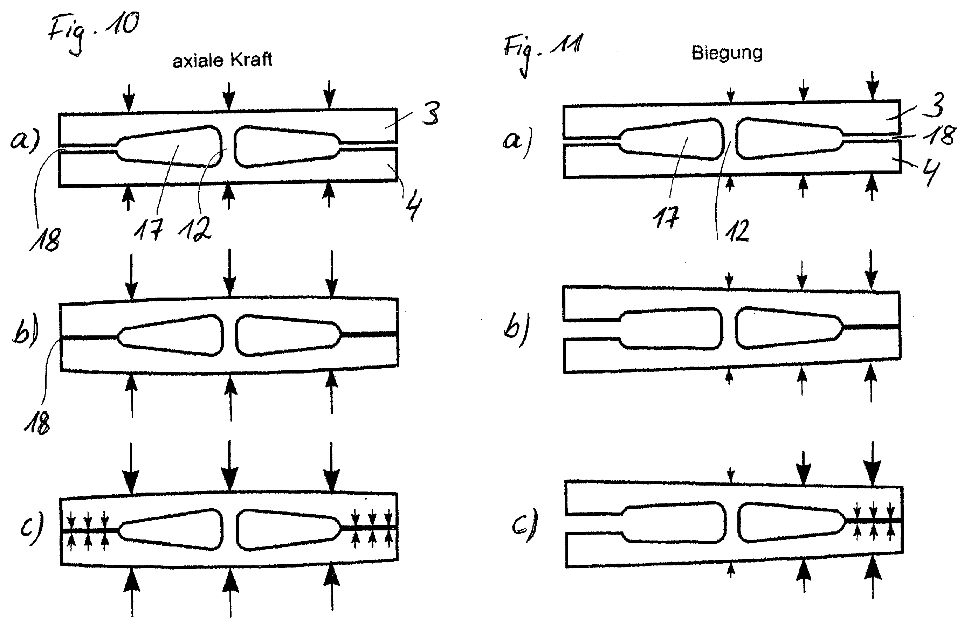

Die Figuren 10 und 11 zeigen die Wirkungsweise der erfindungsgemäßen Struktur.

Wie sich aus der Fig. 10 mit den Teilbildern a) bis c) ergibt, wird bei Aufbringung einer axialen Druckkraft entlang der primären Lastachse L das Filmscharnier, beispielsweise Filmscharnier 12, bzw. die entsprechenden Stege elastisch komprimiert. Zusätzlich, oder bei entsprechender Auslegung der Stege und der Scheiben bzw. Ringe überwiegend werden die Scheiben oder Ringe 3, 4 im Bereich ihrer schwächsten Ausbildung, also in der Nähe des Stegs 12 elastisch verformt bzw. verbogen, bis die beabstandeten Scheibenelemente 3 und 4 im Bereich der Schlitzausbildung 18 aufeinander stoßen. In diesem, im Teilbild b) der Fig. 10 gezeigten Fall wird die axiale Traglast nicht nur von den Stegen des Filmscharniers 12 aufgenommen, sondern auch von den aufeinander liegenden Randbereichen der Scheibenelemente 3 und 4 übernommen. Auf diese Weise wird die aufnehmbare axiale Traglast erhöht. Wird, wie im Teilbild c) der Fig. 10 dargestellt, die axiale Traglast, also die Druckkraft weiter erhöht, so werden die Scheibenelemente 3 und 4 im Bereich der Schlitzausbildung 18 weiter aufeinander gepresst und übernehmen somit die Haupttraglast, da nunmehr die Auflagefläche viel größer ist als die Querschnittsfläche der Stege des Filmscharniers 12.

Bei einer Biegebelastung, wie sie in der Fig. 11 dargestellt ist, ermöglichen die dünn ausgebildeten Stege des Filmscharniers 12 eine Drehung um die Achse senkrecht zur Bildebene. Auf dieses Weise kommt es zu einer einseitigen Anlage der Randbereiche der Scheibenelemente 3 und 4 im Bereich der Schlitzausbildung 18 und einer entsprechenden Verbiegung des Platzhalters bzw. Verkippung der Enden des Platzhalters zueinander. Nach dieser begrenzten Verkipp- bzw. Verbiegemöglicheit kommt es durch die Anlage der Scheibenelemente 3 und 4 aneinander im Bereich der Schlitzausbildung (siehe Teilbild b) der Fig. 11) zu einer Lastübernahme im Bereich der Schlitzausbildung, so dass eine weitere Verkippung verhindert wird und zudem mit zunehmender Lastaufbringung eine Stabilisierung durch einen entsprechend größeren Auflagebereich (Teilbild c) der Fig. 11) eintritt.

Entsprechend ist aus den Figuren 10 und 11 ersichtlich, dass eine entsprechende Gestaltung der Ausnehmungen 17 und 18 zwischen den Scheiben- bzw. Ringelementen 3 und 4 eine Einstellung des möglichen Verkippungs- bzw. Verbiegungsbereichs sowie der Stabilität und Festigkeit des gesamten Implantats ermöglicht. Wird der Anteil des Schlitzbereichs insgesamt vergrößert, so ergibt sich eine höhere Festigkeit, da dann mehr Auflagefläche vorhanden ist. Wird die Schlitzdicke, also der Abstand der Scheiben, vergrößert, erhöht sich die Verbiegbarkeit.

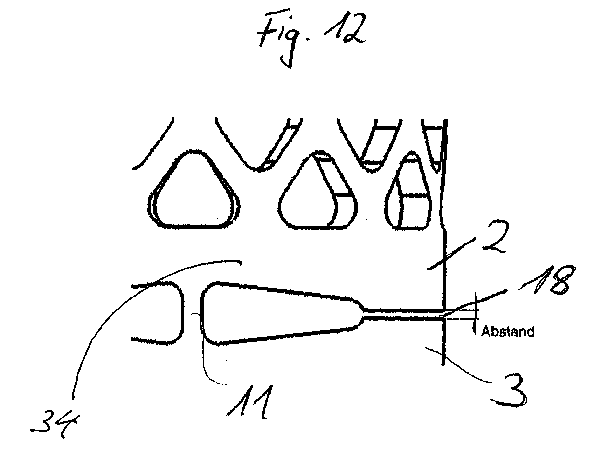

Die Figur 12 verdeutlicht noch einmal im Detail die Wirkung des Schlitzbereichs zwischen den Ring- bzw. Scheibenelementen 2 und 3. Durch den Abstand, der durch den Schlitz 18 zwischen Ring- bzw. Scheibenelementen 2 und 3 definiert ist, wird der Grad der Beweglichkeit bzw. der Verkippung zwischen den Ring- bzw. Scheibenelementen 2 und 3 bestimmt. Je größer der Abstand ist, desto größer ist die Verdrehmöglichkeit der Scheiben- bzw. Ringelemente 2 und 3 bezüglich des Filmscharniers 11. Außerdem bestimmt die Größe der möglichen Auflagefläche im Schlitzbereich 18 die Größe der übertragbaren Last im Falle der axialen Stauchung bzw. entsprechender Biegung. Die Ausbildung des Ring- oder Scheibenelements 2 im Bereich 34 bestimmt die Verbiegbarkeit der Scheibe oder des Rings 2 und somit die Elastizität in Richtung der Lastachse L.

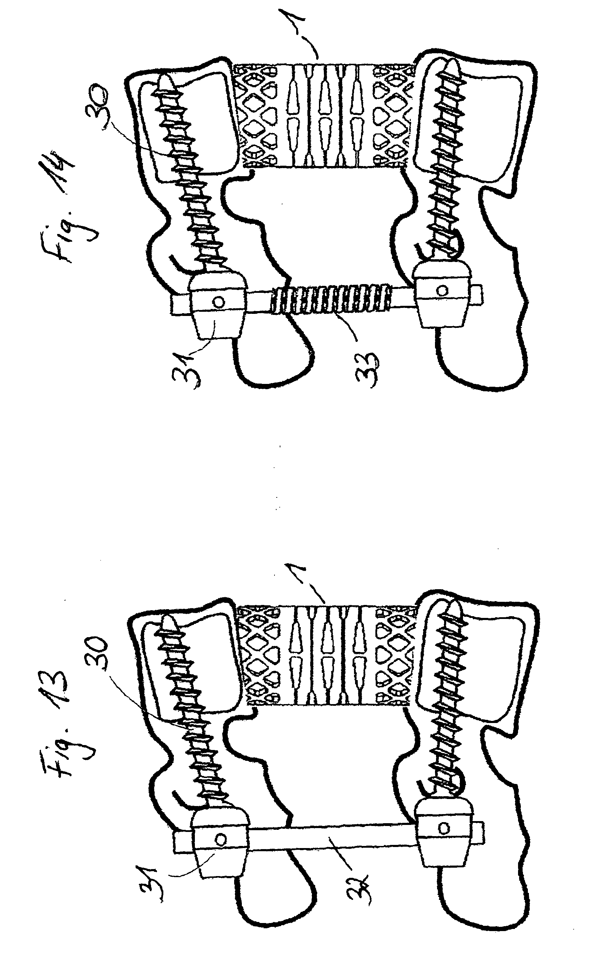

Die Figuren 13 bis 16 zeigen verschiedene Einsatzgebiete erfindungsgemäßer Implantate. In Fig. 13 ist der Platzhalter 1 in Kombination mit einer Pedikelschraubenanordnung zu sehen, wobei die Pedikelschrauben 30 in den benachbarten Wirbelkörpern an Ihren Schraubenköpfen 31 über einen Verbindungsstab 32 verbunden sind und eine Stabilisierungsfunktion ausüben. In diesem Ausführungsbeispiel ist der Verbindungsstab 32 über seine durch das Material implizierte Elastizität halb steif ausgebildet.

Die Fig. 14 zeigt den Platzhalter 1 in einer ähnlichen Anwendungssituation, wobei lediglich in der Pedikelschraubenandordnung der halb steife Verbindungsstab 32 durch einen flexiblen Verbindungsstab 33 ersetzt ist, der in diesem Fall seine Flexibilität durch eine schraubenfederartige Konstruktion mit einem Kernelement erhält. Alternativ könnte zum Verbindungsstab 33 auch ein Verbindungsstab mit der erfindungsgemäßen Struktur aus drehgelenkig miteinander verbundenen Scheiben eingesetzt werden.

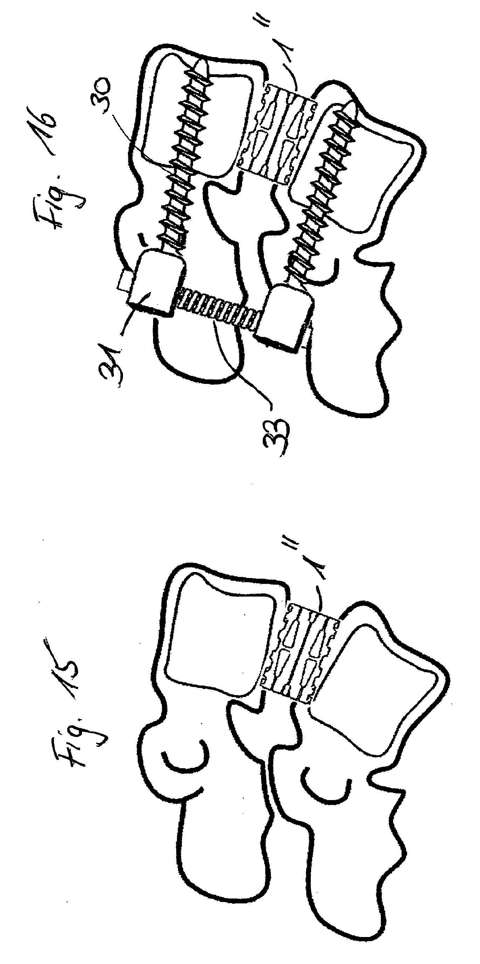

Die Figuren 15 und 16 zeigen das Einsatzgebiet des Platzhalters 1", einmal mit Stabilisierungseinrichtung (Fig. 16) und einmal ohne (Fig. 15). Aufgrund der geringeren Bauhöhe des Platzhalters 1" wird hier der Platzhalter als Bandscheibenersatz eingesetzt, wobei wiederum eine stabilisierende Pedikelschraubenanordnung mit Pedikelschrauben 30 und einem elastischen Verbindungsstab 33, der an den Schraubenköpfen 31 arretiert ist, vorgesehen sein kann.

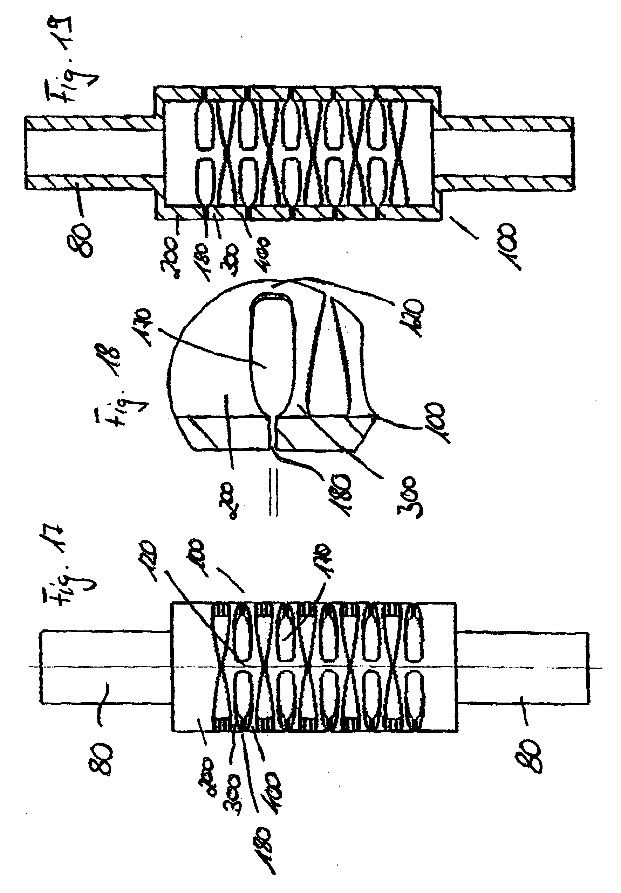

Die Figur 17 zeigt einen flexiblen Verbindungsstab 100, der in einem mittleren Bereich eine den Platzhalter 1 entsprechende erfindungsgemäße Grundkörperstruktur mit gelenkig verbundenen Scheiben- oder Ringelementen 200, 300, 400 umfasst, wobei die Gelenke abwechselnd um 90° zueinander versetzt sind.

Die Gelenke werden durch Filmscharniere 120 gebildet, die beispielsweise durch entsprechende Verbindungsstege zwischen den Ringelementen 200, 300,400 ausgebildet sind, wobei die Breite der Stege 120 der Wanddicke der Ringe 200,300,400 entspricht.

An den axialen Enden des Verbindungsstabs 100 sind Verbindungsstutzen 80 zur Aufnahme in z. B Schraubköpfen von Pedikelschrauben vorgesehen. Bei der in der Figur 17 gezeigten Ausführungsform weisen die Verbindungsstutzen 80 einen geringeren Querschnitt auf, als der Mittelbereich mit der Gelenkstruktur. Allerdings sind auch Stäbe vorstellbar, die einen durchgehend gleichen Querschnitt aufweisen.

Die Figur 18 zeigt in einer Detailansicht des Verbindungsstabes 100 aus Figur 17, dass hier die entsprechenden Ringelemente 200 und 300 ebenfalls nur durch entsprechende Stegelemente 120 miteinander verbunden sind, während im übrigen Wandbereich zwischen den Ringen 200,300 eine flaschenförmige Ausnehmung 170, 180 mit einem breiten Ausnehmungsbereich 170 und einem schlitzförmigen Ausnehmungsbereich 180 vorgesehen ist. Durch die Gestalt der Ausnehmungen 170, 180 und insbesondere die Dicke des Schlitzes 180 wird wiederum die Komprimierbarkeit sowie die Kipp- bzw. Biegbarkeit des Verbindungsstabs 100 definiert.

Die Figur 19 zeigt eine Schnittansicht des Verbindungsstabs 100 der Figuren 17 und 18. Hier wird deutlich, dass die Verbindungsstutzen 80 einstückig mit dem Grundkörper ausgebildet sind. Allerdings ist es auch möglich, die Verbindungsstutzen 80 in geeigneter Weise lösbar mit der Grundstruktur zu verbinden, beispielsweise durch entsprechende Schraubverbindungen.

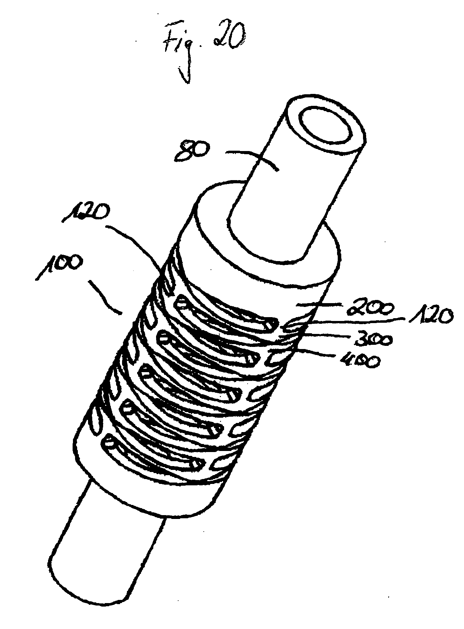

Die Figur 20 zeigt den flexiblen Verbindungsstab 100 noch einmal in perspektivischer Darstellung wobei deutlich wird, dass die zylinderrohrförmige Grundstruktur mit über Stege bzw. Filmscharniere 120 gelenkig verbundenen Ringelementen 200, 300,400 aufgebaut ist, wobei die Stege bzw. die Filmscharniere benachbarter Ringelemente jeweils um 90° zueinander verdreht sind.

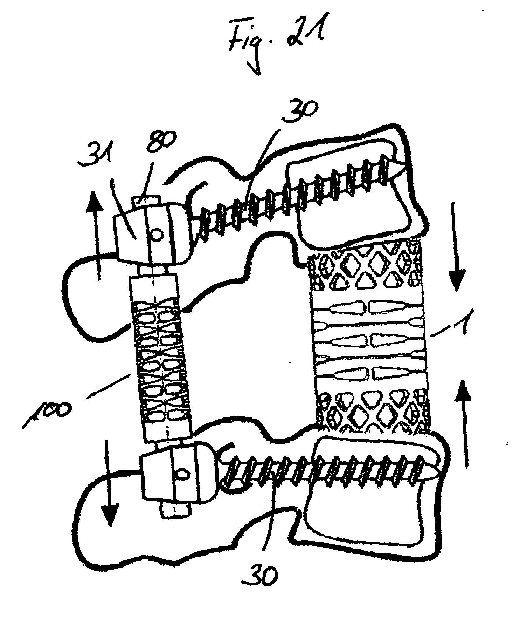

Die Figur 21 zeigt einen Anwendungsfall für den erfindungsgemäßen Platzhalter 1 und den flexiblen Verbindungsstab 100 sowie ein Stabilisierungssystem einer Pedikelschraubenanordnung. Die in den Wirbelkörpern eingeschraubten Pedikelschrauben 30 sind über den flexiblen Verbindungsstab 100 miteinander verbunden, wobei die Verbindungsstutzen 80 von den Schraubenköpfen 31 aufgenommen sind. Zwischen den Wirbelkörpern ist der Platzhalter 1 vorgesehen. Durch sowohl die flexible Ausbildung des Platzhalters 1 als auch des Verbindungsstabs 100 ist beispielsweise, wie durch die Pfeile in der Figur 21 angedeutet, eine Komprimierung des Platzhalters 1 bei gleichzeitiger Dehnung des flexiblen Verbindungsstabs 100 bzw. umgekehrt möglich. Zudem können sowohl der flexible Verbindungsstab 100 als auch der flexible Platzhalter 1 entsprechende Biegungen bzw. Verkippungen ihrer axialen Enden durchführen. Insgesamt können somit die gewünschten Anforderungen hinsichtlich der Festigkeit einerseits und Flexibilität andererseits in idealer Weise erfüllt werden.

## Patentansprüche

1. Implantat zur dauerhaften oder zeitweisen Verbringung in den menschlichen oder tierischen Körper mit einem rohrförmigen Grundkörper zur Verbindung beabstandeter Körperteile und/oder anderer Implantatkomponenten, welcher eine Lastachse (L) aufweist, entlang der primär Zug- und/oder Druckkräfte übertragbar sind, wobei quer zur Lastachse mindestens eine Drehachse (D) ausgebildet ist, welche ein zumindest begrenztes Biegen des Grundkörpers um die entlang der Lastachse angeordneten Enden des Grundkörpers, ermöglicht, wobei die mindestens eine Drehachse durch ein Drehgelenk (11, 12, 13, 14, 15) definiert ist, welches einstückig am Grundkörper ausgebildet ist, **dadurch gekennzeichnet, dass**
mehrere Drehgelenke (11, 12, 13, 14, 15) mit ihren Drehachsen in mehreren entlang der Lastachse übereinander liegenden Ebenen quer zur Lastachse (L) und gegenseitig um die Lastachse (L) verdrehten Drehachsen vorgesehen sind, wobei alternierend die Drehgelenke zueinander um 90° versetzt sind, und wobei die Drehgelenke mehrere über einander angeordnete Scheiben- oder Ringelemente miteinander verbinden und dass jedes Drehgelenk (11, 12, 13, 14, 15) in Form eines Filmscharniers ausgebildet ist, wobei das Drehgelenk mindestens einen entlang einer Halbierenden der einzelnen Scheiben- oder Ringelemente radial von der äußeren Umfangswand des rohrförmigen Grundkörpers nach innen in Richtung der zur Lastachse parallelen Mittelachse des GrundKörpers verlaufenden Steg umfasst, an dem beidseits Aussparungen vorgesehen sind.

2. Implantat nach Anspruch 1,
**dadurch gekennzeichnet, dass**
mehrere Drehgelenke (11, 12, 13, 14, 15) mit Ihren Drehachsen In unterschiedlichen Ebenen senkrecht zur Lastachse (L) angeordnet sind.

3. Implantat nach einem der vorhergehenden Ansprüche,
**dadurch gekennzeichnet, dass**
das Drehgelenk (11, 12, 13, 14, 15) durch zwei sich entlang der Drehachse gegenüberliegende, sich in einer Ebene parallel zur Lastachse (L) erstreckende Stege gebildet ist.

4. Implantat nach einem der vorhergehenden Ansprüche,
**dadurch gekennzeichnet, dass**
der Grundkörper In einer Ebene senkrecht zur Lastachse (L), In der ein Drehgelenk in Form eines senkrecht zu dieser Ebene vorgesehenen Stegs, angeordnet ist, zwei auf beiden Seiten des Drehgelenks angeordnete Ausnehmungen (17, 18) aufweist, die ein Kippen um das Drehgelenk ermöglichen.

5. Implantat nach Anspruch 4
**dadurch gekennzeichnet, dass**
die Ausnehmung (17, 18) sich ausgehend von dem Drehgelenk (11, 12, 13, 14, 15) zumindest im Wandbereich des Grundkörpers verjüngt.

6. Implantat nach einem der vorhergehenden Ansprüche,
**dadurch gekennzeichnet, dass**
der Grundkörper und insbesondere die von diesem umfassten Scheiben oder Ringe (2, 3, 4, 5, 6, 7) eine zentrale, koaxial zur Mittelachse angeordnete, entlang der Lastachse durchgehende Öffnung (19 bis 24) aufweisen.

7. Implantat nach Anspruch 6,
**dadurch gekennzeichnet, dass**
die Öffnung (19 bis 24) stem-, kreuz- oder kleeblattförmig ist.

8. Implantat nach einem der vorhergehenden Ansprüche,
**dadurch gekennzeichnet, dass**
vier bis zehn, vorzugsweise vier bis sechs Scheiben oder Ringe (2, 3, 4, 5, 6, 7) vorgesehen sind.

9. Implantat nach einem der vorhergehenden Ansprüche,
**dadurch gekennzeichnet, dass**
drei bis fünf Drehgelenke (11, 12, 13, 14, 15) vorgesehen sind.

10. Implantat nach einem der vorhergehenden Ansprüche,
**dadurch gekennzeichnet, dass**
der Grundkörper eine ungerade Anzahl von Drehgelenken (11, 12, 13, 14, 15) und eine gerade Anzahl von Scheiben oder Ringen (2 bis 7) aufweist.

11. Implantat nach einem der vorhergegangenen Ansprüche,
**dadurch gekennzeichnet, dass**
der Grundkörper in axialer Richtung parallel zur Lastachse elastisch verformbar, insbesondere komprimierbar ist.

12. Implantat nach einem der vorhergegangenen Ansprüche,
**dadurch gekennzeichnet, dass**
die vom Grundkörper umfassten Scheiben oder Ringe (2, 3, 4, 5, 6, 7) derart ausgebildet sind, dass überwiegend sie bel axialer Last entlang der Lastachse, Insbesondere Drucklast elastisch verformt, insbesondere in der Nähe der Drehgelenke verbogen werden.

13. Implantat nach einem der vorhergehenden Ansprüche,
**dadurch gekennzeichnet, dass**
der Grundkörper als rohrartiger Körper mit beliebigen Querschnittsformen, insbesondere als zylinderrohrförmiger Körper ausgebildet ist.

14. Implantat nach einem der vorhergehenden Ansprüche,
**dadurch gekennzeichnet, dass**
der Grundkörper an seinen entlang der Lastachse angeordneten Enden Verbindungselemente (8, 9, 10) aufweist.

15. Implantat nach einem der vorhergehenden Ansprüche,
**dadurch gekennzeichnet, dass**
das Implantat ein Platzhalter für Wirbelkörper, ein Bandscheibenersatz oder ein Verbindungsstab für Pedlkelschraubenanordnungen oder sonstige Stabilisierungseinrichtungen ist.

16. Implantat nach Anspruch 14,
**dadurch gekennzeichnet, dass**
die Verbindungselemente (8, 9, 10) durch Durchbrüche, Ausnehmungen, Vorsprünge, Zacken zum Eingreifen oder Einwachsen in benachbartes Körpergewebe und/oder durch in Schraubverbindungen einklemmbare Stutzen oder dergleichen gebildet sind.

## Claims

1. Implant, for permanent or temporary implantation into a human or animal body, having a tubular base body for connecting spaced body parts and/or other implant components, said implant having a load axis (L) along which predominantly tensile and/or compressive forces can be transmitted, wherein, transverse to the load axis is formed at least one rotary axis (D), which facilitates at least limited bending of the base body about the ends of the base body which are arranged along the load axis, wherein the at least one rotary axis is defined by a swivel joint (11, 12, 13, 14, 15), which is formed monolithically at the base body,
**characterised by** the fact that
several swivel joints (11, 12, 13, 14, 15) with their rotary axes are provided along the load axis in several stacked planes transverse to the load axis (L) and with rotary axes rotated mutually about the load axis (L), wherein the swivel joints are offset from each other at alternating angles of 90°, and wherein the swivel joints connect several stacked arrangements of discs or ring elements together and that each swivel joint (11, 12, 13, 14, 15) is formed in the manner of a film hinge, wherein the swivel joint comprises at least one strut, which has cutouts on either side and which proceeds radially inwards, from the outer circumferential wall of the tubular base body, along the bisector of the individual discs or ring elements, towards the centre axis, which is parallel with the load axis.

2. Implant in accordance with claim 1,
**characterised by** the fact that
several swivel joints (11, 12, 13, 14, 15) with their rotary axes are arranged in different planes perpendicularly to the load axis (L).

3. Implant in accordance with any of the previous claims,
**characterised by** the fact that
the swivel joint (11, 12, 13, 14, 15) is formed by two struts which extend opposite each other on the rotary axis in a plane parallel with the load axis (L).

4. Implant in accordance with any of the previous claims,
**characterised by** the fact that
the base body has, in a plane perpendicular to the load axis (L) in which is arranged a swivel joint in the form of a strut provided perpendicularly to this plane, two cutouts (17, 18) which are arranged on both sides of the swivel joint and which facilitate tilting about the swivel joint.

5. Implant in accordance with claim 4,
**characterised by** the fact that
the cutout (17, 18), proceeding from the swivel joint (11, 12, 13, 14, 15), tapers at least in the wall area of the base body.

6. Implant in accordance with any of the previous claims,
**characterised by** the fact that
the base body, and especially the discs or rings (2, 3, 4, 5, 6, 7) which it comprises, have a central, continuous aperture (19 to 24) which is coaxial with the centre axis and which extends along the load axis.

7. Implant in accordance with claim 6,
**characterised by** the fact that
the aperture (19 to 24) has the shape of a star, cross or clover leaf.

8. Implant in accordance with any of the previous claims,
**characterised by** the fact that
four to ten, preferably four to six discs or rings (2, 3, 4, 5, 6, 7) are provided.

9. Implant in accordance with any of the previous claims,
**characterised by** the fact that
three to five swivel joints (11, 12, 13, 14, 15) are provided.

10. Implant in accordance with any of the previous claims,
**characterised by** the fact that
the base body has an odd number of swivel joints (11, 12, 13, 14, 15) and an even number of discs or rings (2 to 7).

11. Implant in accordance with any of the previous claims,
**characterised by** the fact that
the base body is elastically deformable, especially compressible, in the axial direction parallel to the load axis.

12. Implant in accordance with any of the previous claims,
**characterised by** the fact that
the discs or rings (2, 3, 4, 5, 6, 7) which the base body comprises are formed such that predominantly they deform elastically, especially are bent in the vicinity of the swivel joints, by axial load along the load axis, especially compressive load.

13. Implant in accordance with any of the previous claims,
**characterised by** the fact that
the base body is formed as a tubular body with arbitrary cross-sectional shapes, especially as a cylindrical-tubular body.

14. Implant in accordance with any of the previous claims,
**characterised by** the fact that
the ends of the base body, which are arranged along the load axis, have connecting elements (8, 9, 10).

15. Implant in accordance with any of the previous claims,
**characterised by** the fact that
the implant is formed as a placeholder for vertebrae, as replacement for vertebral discs or as a connecting rod for pedicle screw arrangements or other stabilising devices.

16. Implant in accordance with claim 14,
**characterised by** the fact that
the connecting elements (8, 9, 10) are formed by breakthroughs, cutouts, projections, serrations for engaging with or knitting with adjacent body tissue and/or by supports or the like which can be clamped into screw connections.

## Revendications

1. Implant pour une pose permanente ou temporaire dans le corps humain ou animal avec un corps de base tubulaire pour relier des parties de corps espacées et/ou d'autres composants d'implant, qui présente un axe de charge (L), le long duquel des forces de tension et/ou de pression primaires peuvent être transmises, au moins un axe de rotation (D) étant configuré transversalement par rapport à l'axe de charge, axe qui permet une flexion tout au moins limitée du corps de base autour des extrémités placées le long de l'axe de charge, l'axe de rotation qui existe au moins étant défini par une articulation tournante (11, 12, 13, 14, 15) qui est configurée en une pièce sur le corps de base,
**caractérisé en ce**
**que** plusieurs articulations tournantes (11, 12, 13, 14, 15) sont prévues avec leurs axes de rotation dans plusieurs plans superposés le long de l'axe de charge transversalement par rapport à l'axe de charge (L) et tordus l'un contre l'autre autour de l'axe de charge (L), les articulations tournantes étant décalées en alternance de 90° l'une par rapport à l'autre et les articulations tournantes reliant plusieurs éléments de disque ou d'anneau placés superposés et que chaque articulation tournante (11, 12, 13, 14, 15) est configurée en forme de charnière souple, l'articulation tournante comprenant au moins une barrette le long d'une bissectrice des différents éléments de disque ou d'anneau radialement à partir de la paroi périphérique extérieure du corps de base tubulaire vers l'intérieur en direction de l'axe central du corps de base parallèle à l'axe de charge, barrette sur laquelle des évidements sont prévus des deux côtés.

2. Implant selon la revendication 1,
**caractérisé en ce**
**que** plusieurs articulations tournantes (11, 12, 13, 14, 15) sont placées avec leurs axes de rotation dans différents plans perpendiculairement à l'axe de charge (L).

3. Implant selon l'une des revendications précédentes,
**caractérisé en ce**
**que** l'articulation tournante (11, 12, 13, 14, 15) est formée par deux barrettes en face l'une de l'autre le long de l'axe de rotation qui s'étendent dans un plan parallèlement à l'axe de charge (L).

4. Implant selon l'une des revendications précédentes,
**caractérisé en ce**
**que** le corps de base présente, dans un plan perpendiculairement à l'axe de charge (L), dans lequel une articulation tournante en forme de barrette prévue perpendiculairement à ce plan est placée, deux évidements (17, 18) placés des deux côtés de l'articulation tournante qui permettent un basculement autour de l'articulation tournante.

5. Implant selon la revendication 4,
**caractérisé en ce**
**que** l'évidement (17, 18) s'effile en partant de l'articulation tournante (11, 12, 13, 14, 15) au moins dans la zone de la paroi du corps de base.

6. Implant selon l'une des revendications précédentes,
**caractérisé en ce**
**que** le corps de base et en particulier les disques ou anneaux (2, 3, 4, 5, 6, 7) ceints par celui-ci présentent une ouverture centrale (19 à 24) placée coaxiale avec l'axe central, continue le long de l'axe de charge.

7. Implant selon la revendication 6,
**caractérisé en ce**
**que** l'ouverture (19 à 24) est en forme d'étoile, de croix ou de feuille de trèfle.

8. Implant selon l'une des revendications précédentes,
**caractérisé en ce**
**que** quatre à dix, de préférence quatre à six, disques ou anneaux (2, 3, 4, 5, 6, 7) sont prévus.

9. Implant selon l'une des revendications précédentes,
**caractérisé en ce**
**que** trois à cinq articulations tournantes (11, 12, 13, 14, 15) sont prévues.

10. Implant selon l'une des revendications précédentes,
**caractérisé en ce**
**que** le corps de base présente un nombre impair d'articulations tournantes (11, 12, 13, 14, 15) et un nombre pair de disques ou d'anneaux (2 à 7).

11. Implant selon l'une des revendications précédentes,
**caractérisé en ce**
**que** le corps de base est déformable élastiquement dans le sens axial parallèlement à l'axe de charge, en particulier qu'il peut être comprimé.

12. Implant selon l'une des revendications précédentes,
**caractérisé en ce**
**que** les disques ou anneaux (2, 3, 4, 5, 6, 7) ceints par le corps de base sont formés de telle manière que la plupart du temps, en cas de charge axiale le long de l'axe de charge, en particulier de charge de pression, ils sont déformés élastiquement, en particulier qu'ils sont gauchis à proximité des articulations tournantes.

13. Implant selon l'une des revendications précédentes,
**caractérisé en ce**
**que** le corps de base est configuré comme un corps de type tube avec des formes de section quelconques, en particulier qu'il est configuré comme un corps en forme de tube cylindrique.

14. Implant selon l'une des revendications précédentes,
**caractérisé en ce**
**que** le corps de base présente des éléments de liaison (8, 9, 10) à ses extrémités placées le long de l'axe de charge.

15. Implant selon l'une des revendications précédentes,
**caractérisé en ce**
**que** l'implant est un garde-place pour un corps de vertèbre, un remplacement de disque intervertébral ou une tige de liaison pour des arrangements de vis pédiculaires ou d'autres dispositifs de stabilisation.

16. Implant selon la revendication 14,
**caractérisé en ce**
**que** les éléments de liaison (8, 9, 10) sont formés par des percements, des évidements, des saillies, des dents pour l'engrènement ou la croissance dans le tissu voisin du corps et/ou par des étais ou équivalents qui peuvent être serrés dans des assemblages vissés.
